# DEMANDE DE BREVET EUROPEEN

(11) **EP 4 304 050 A1**
(43) Date de publication de la demande: **10.01.2024**
(21) Numéro de dépôt: 23180522.7
(22) Date de dépôt: 21.06.2023
(51) Int. Cl.: H02J 50/15

(54) **SYSTEME DE RECHARGE D'UN DISPOSITIF MEDICAL IMPLANTABLE**

(30) Priorité: 05.07.2022 FR 2206806
(71) Demandeur: Vermon, 37000 Tours (FR)
(72) Inventeur: ROSINSKI, Bogdan, 37000 TOURS (FR); HOANG, Thien, 37000 TOURS (FR); FELIX, Nicolas, 37000 TOURS (FR)
(74) Mandataire: Cabinet Beaumont

(57) **Abrégé**

La présente description concerne un dispositif (200) d'émission d'ultrasons pour la recharge sans fil d'un dispositif électronique, le dispositif d'émission comprenant au moins un transducteur ultrasonore (201), et un circuit électronique (203, 205, 207) de contrôle du transducteur (201), le circuit électronique de contrôle (203, 205, 207) étant configuré pour :
- appliquer au transducteur (201) un signal électrique d'excitation de façon à provoquer l'émission d'une onde ultrasonore en direction du dispositif électronique ;
- lire un signal électrique retour généré par le transducteur (201) sous l'effet d'une portion de l'onde ultrasonore réfléchie par le dispositif électronique,
le circuit électronique de contrôle (203, 205, 207) comprenant une boucle de rétroaction (205, 207) configurée pour ajuster la fréquence de l'onde ultrasonore de façon à faire tendre vers une valeur minimale l'énergie de la portion réfléchie de l'onde ultrasonore.

## Description

### Domaine technique

La présente description concerne de façon générale les dispositifs et systèmes à base de transducteurs ultrasonores, et vise plus particulièrement un système de recharge sans fil, par ultrasons, d'un dispositif médical implantable.

### Technique antérieure

Un dispositif médical implantable est un dispositif destiné à être implanté dans le corps d'un patient pour surveiller et/ou traiter divers types de pathologies. Un tel dispositif peut comprendre un ou plusieurs capteurs, par exemple pour mesurer un ou plusieurs paramètres physiologiques, et/ou un ou plusieurs actionneurs, par exemple pour administrer un traitement ou stimuler un organe.

Ces éléments sont généralement logés dans un boîtier en un matériau biocompatible, que l'on vient implanter dans le corps du patient.

Un dispositif médical implantable comprend classiquement une batterie électrique non rechargeable pour alimenter ses différents composants. Ceci présente toutefois des limitations en termes de durée de vie et d'encombrement.

Pour réduire l'encombrement et/ou augmenter la durée de vie d'un dispositif médical implantable, il a été proposé d'utiliser une batterie rechargeable alimentée par un système de transfert d'énergie sans fil, et, plus particulièrement, par un système de transfert d'énergie électrique sans fil par ultrasons.

Il serait souhaitable d'améliorer au moins en partie certains aspects des solutions connues de recharge sans fil, par ultrasons, de dispositifs médicaux implantables.

### Résumé de l'invention

Pour cela, un mode de réalisation prévoit un dispositif d'émission d'ultrasons pour la recharge sans fil d'un dispositif électronique, le dispositif d'émission comprenant au moins un transducteur ultrasonore, et un circuit électronique de contrôle dudit au moins un transducteur ultrasonore, le circuit électronique de contrôle étant configuré pour :
- appliquer au transducteur ultrasonore un signal électrique d'excitation de façon à provoquer l'émission, par le transducteur ultrasonore, d'une onde ultrasonore en direction du dispositif électronique ;
- lire un signal électrique retour généré par le transducteur ultrasonore sous l'effet d'une portion de l'onde ultrasonore réfléchie par le dispositif électronique,
le circuit électronique de contrôle comprenant une boucle de rétroaction configurée pour ajuster la fréquence de l'onde ultrasonore de façon à faire tendre vers une valeur minimale l'énergie de la portion réfléchie de l'onde ultrasonore.

Selon un mode de réalisation, le circuit électronique de contrôle est configuré pour appliquer au transducteur ultrasonore un signal électrique d'excitation modulé en fréquence dont la fréquence varie de façon continue entre une fréquence fstart et une fréquence fstop, supérieure à fstart.

Selon un mode de réalisation, la boucle de rétroaction comprend un circuit de traitement adapté à détecter la fréquence fNZR à laquelle l'énergie de la portion réfléchie de l'onde ultrasonore est minimale.

Selon un mode de réalisation, le circuit de traitement est en outre configuré pour ajuster les valeurs des fréquences fstart et fstop de façon à les rapprocher de la fréquence fNZR, et ainsi réduire l'énergie de la portion réfléchie de l'onde ultrasonore.

Selon un mode de réalisation, le circuit de contrôle comprend un circuit de détection de l'enveloppe dudit signal électrique retour généré par le transducteur ultrasonore.

Selon un mode de réalisation, le circuit de contrôle comprend un circuit de détection de fronts de l'enveloppe du signal électrique retour généré par le transducteur ultrasonore.

Selon un mode de réalisation, le circuit électronique de contrôle est configuré pour appliquer au transducteur ultrasonore un signal électrique d'excitation impulsionnel.

Selon un mode de réalisation, le circuit électronique de contrôle est configuré pour appliquer au transducteur ultrasonore un signal électrique d'excitation continu.

Selon un mode de réalisation, le transducteur ultrasonore comprend un transducteur d'émission et un transducteur de réception.

Selon un mode de réalisation, le transducteur ultrasonore comprend un transducteur unique pour l'émission de l'onde ultrasonore et pour la réception de la portion réfléchie de l'onde ultrasonore.

### Brève description des dessins

Ces caractéristiques et avantages, ainsi que d'autres, seront exposés en détail dans la description suivante de modes de réalisation particuliers faite à titre non limitatif en relation avec les figures jointes parmi lesquelles :
la figure 1 est un diagramme représentant de façon schématique le rendement de conversion d'énergie d'un transducteur ultrasonore en fonction de la fréquence acoustique de l'onde reçue et de la charge électrique du transducteur ;
la figure 2 représente de façon schématique, sous forme de blocs, un exemple d'un dispositif d'émission d'ultrasons pour la recharge sans fil d'un dispositif médical implantable ; et
la figure 3 est un diagramme illustrant un exemple de fonctionnement du dispositif de la figure 2.

### Description des modes de réalisation

De mêmes éléments ont été désignés par de mêmes références dans les différentes figures. En particulier, les éléments structurels et/ou fonctionnels communs aux différents modes de réalisation peuvent présenter les mêmes références et peuvent disposer de propriétés structurelles, dimensionnelles et matérielles identiques.

Par souci de clarté, seuls les étapes et éléments utiles à la compréhension des modes de réalisation décrits ont été représentés et sont détaillés. En particulier, la réalisation des transducteurs ultrasonores et des circuits électroniques de contrôle des dispositifs décrits n'a pas été détaillée, les modes de réalisation décrits étant compatible avec les réalisations usuelles de ces éléments ou la réalisation de ces éléments étant à la portée de la personne du métier à partir des indications de la présente description.

Sauf précision contraire, lorsque l'on fait référence à deux éléments connectés entre eux, cela signifie directement connectés sans éléments intermédiaires autres que des conducteurs, et lorsque l'on fait référence à deux éléments reliés (en anglais "coupled") entre eux, cela signifie que ces deux éléments peuvent être connectés ou être reliés par l'intermédiaire d'un ou plusieurs autres éléments.

Sauf précision contraire, les expressions "environ", "approximativement", "sensiblement", et "de l'ordre de" signifient à 10 % près, de préférence à 5 % près.

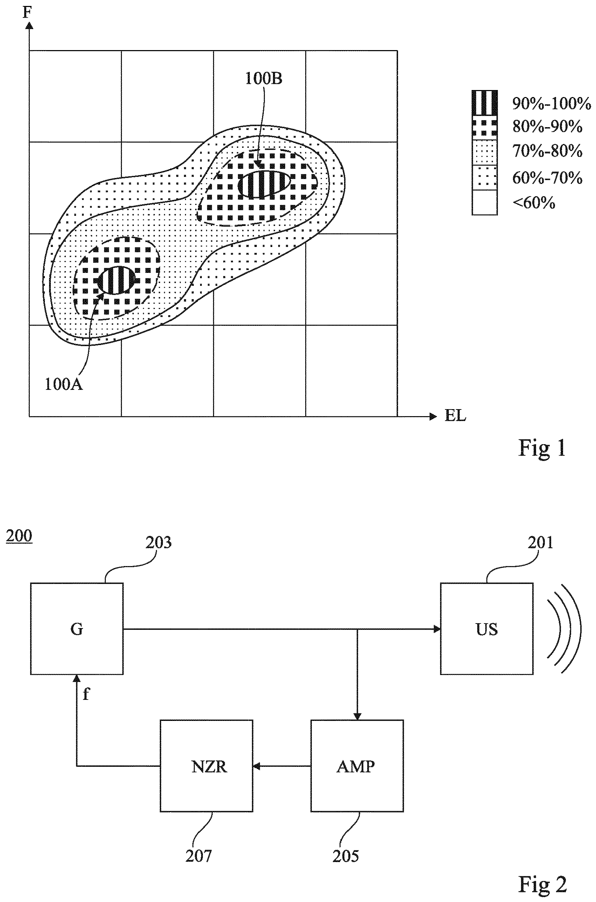

La figure 1 est un diagramme représentant de façon schématique le rendement de conversion d'énergie d'un transducteur ultrasonore (par plages de pourcentage d'énergie acoustique convertie en énergie électrique, représentées par des texturations différentes), en fonction de la fréquence acoustique F de l'onde reçue par le transducteur, en ordonnée, et de la charge électrique EL vue par le transducteur, en abscisse.

Le transducteur est par exemple intégré dans un dispositif médical implantable (non détaillé) disposé dans le corps d'un patient. Le dispositif médical implantable comprend par exemple un circuit électronique connecté aux bornes du transducteurs et adapté à convertir des signaux électriques générés aux bornes du transducteur sous l'effet d'une onde acoustique reçue, en des signaux électriques d'alimentation ou de recharge d'une batterie électrique du dispositif médical implantable.

L'onde acoustique est par exemple émise par un dispositif d'émission situé à l'extérieur du corps du patient. Ceci permet de mettre en oeuvre une recharge sans fil, par ultrasons, de la batterie électrique du dispositif médical implantable.

Comme l'illustre la figure 1, le rendement de conversion de l'énergie acoustique émise par le dispositif d'émission en énergie électrique d'alimentation du dispositif implantable présente des pics (maximas) pour certaines valeurs de la fréquence acoustique de l'onde reçue, et de la charge électrique connectée au transducteur. Plus particulièrement, dans l'exemple représenté, le rendement de conversion présente deux maximas 100A et 100B correspondant respectivement à un mode de résonance et à un mode d'anti-résonnance du transducteur de réception.

Ces valeurs optimales de fréquence acoustique et de charge électrique peuvent varier d'un dispositif à un autre ou dans le temps, en raison notamment des dispersions de fabrication, et des variations d'impédance acoustique du milieu environnant. Par ailleurs, la charge électrique vue par le transducteur peut varier dans le temps, en fonction notamment de la consommation électrique du dispositif médical implantable.

Ainsi, lors de la mise en oeuvre d'une recharge sans fil par ultrasons d'un dispositif médical implantable, il est souhaitable de pouvoir ajuster dynamiquement la fréquence acoustique d'émission du dispositif externe de façon à maximiser le rendement de la chaîne de transmission d'énergie.

Pour cela, une possibilité est de prévoir un lien de communication, par exemple un lien de communication sans fil, par exemple basé sur des signaux radio-fréquence (RF) ou ultrasonores. Le dispositif implantable peu ainsi transmettre au dispositif externe un retour d'information relatif à la quantité d'énergie électrique reçue. Le dispositif d'émission peut alors ajuster la fréquence d'émission ultrasonore sur la base de cette information, de façon à maximiser le rendement de conversion. Un inconvénient de cette approche réside toutefois dans le surcoût et la surconsommation électrique associés au circuit de communication sans-fil.

La part de l'énergie acoustique émise par le dispositif d'émission et non convertie en énergie électrique par le transducteur intégré au dispositif médical implantable est, au moins pour partie, réfléchie par le dispositif médical implantable, en direction de la source d'émission.

Selon un aspect d'un mode de réalisation, le dispositif d'émission est configuré pour mesurer la portion de l'onde ultrasonore réfléchie par le dispositif médical implantable, et ajuster la fréquence d'émission de façon à faire tendre vers une valeur minimale l'énergie de ladite portion réfléchie de l'onde ultrasonore. Ceci permet de faire tendre vers une valeur maximale le rendement de la chaîne complète de transmission d'énergie électrique vers le dispositif médical implantable.

La figure 2 représente de façon schématique, sous forme de blocs, un exemple d'un dispositif 200 d'émission d'ultrasons pour la recharge sans fil d'un dispositif médical implantable, selon un mode de réalisation.

Le dispositif 200 comprend au moins un transducteur ultrasonore 201 (US) adapté à émettre des ondes ultrasonores en direction du dispositif médical implantable à recharger. Le transducteur 201 est en outre adapté à recevoir des ondes ultrasonores en provenance du dispositif médical implantable. En particulier, le transducteur ultrasonore est adapté, suite à l'émission d'une onde ultrasonore de recharge en direction du dispositif médical implantable, à recevoir une portion de ladite onde réfléchie par le dispositif médical implantable.

De préférence, le transducteur 201 comprend deux transducteurs ultrasonores dédiés respectivement à l'émission d'une onde ultrasonore de recharge en direction du dispositif médical implantable, et à la réception d'une portion de ladite onde réfléchie par le dispositif médical implantable. A titre de variante, l'émission et la réception sont réalisées par un même transducteur ultrasonore.

Le transducteur 201 est par exemple un transducteur piézoélectrique. Plus généralement, les modes de réalisations peuvent être adaptés à tout type de transducteur ultrasonore, par exemple des transducteurs capacitifs à membrane (CMUT), des transducteurs piézoélectriques à membrane (PMUT), etc.

Le dispositif 200 comprend en outre un circuit 203 (G) de génération de signaux électriques d'excitation appliqués au transducteur 201 pour provoquer l'émission de l'onde ultrasonore de recharge par le transducteur 201. Le circuit 203 est en particulier adapté à régler la fréquence des signaux électrique d'excitation générés selon une fréquence de consigne f, de façon à régler la fréquence acoustique de l'onde ultrasonore de recharge émise par le transducteur 201.

Le dispositif 200 comprend en outre une boucle de rétroaction adaptée à lire un signal électrique retour généré par le transducteur 201 sous l'effet de la portion de l'onde ultrasonore de recharge réfléchie par le dispositif médical implantable, et adapter en conséquence la fréquence de consigne du circuit 203, et par conséquent la fréquence d'émission du transducteur 201, de façon à faire tendre vers une valeur minimale l'énergie de la portion réfléchie de l'onde ultrasonore de recharge.

Dans l'exemple représenté, la boucle de rétroaction comprend un amplificateur 205 (AMP) adapté à amplifier le signal électrique retour généré par le transducteur 201 sous l'effet de la portion de l'onde ultrasonore de recharge réfléchie par le dispositif médical implantable.

Dans cet exemple, la boucle de rétroaction comprend en outre un circuit de traitement 207 (NZR) recevant le signal retour amplifié fourni par l'amplificateur 205, et adapté à générer un signal représentatif de l'énergie de la portion réfléchie de l'onde ultrasonore de recharge. Le circuit 207 est adapté à ajuster la consigne de fréquence f fournie au circuit 203 de façon à minimiser l'énergie de la portion réfléchie de l'onde ultrasonore de recharge.

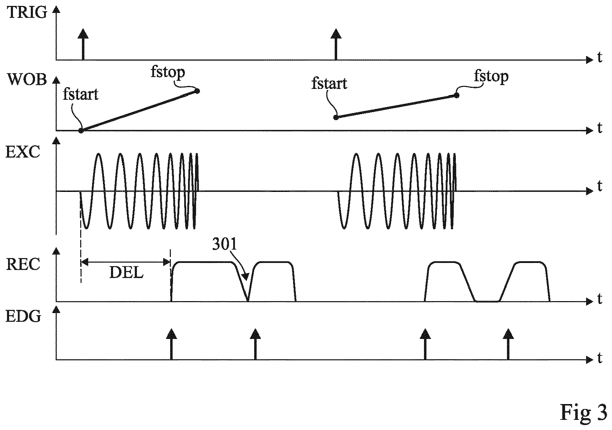

La figure 3 est un diagramme illustrant un exemple de fonctionnement du dispositif 200 de la figure 2.

Dans cet exemple, l'onde acoustique ultrasonore émise par le dispositif d'émission 200 a la forme d'un train d'impulsions (« burst waves »). A titre d'exemple, la fréquence de répétition des impulsions est fixe, et le rapport cyclique des impulsions (ou la durée des impulsions) est fixe.

La figure 3 représente plus particulièrement l'évolution, en fonction du temps t (en abscisse) :
- d'un signal TRIG de déclenchement des impulsions,
- d'un signal WOB représentatif de la fréquence de consigne appliquée au circuit 203 de génération du signal d'excitation du transducteur 201,
- du signal d'excitation EXC généré par le circuit 203,
- d'un signal REC représentatif de l'enveloppe de la portion de l'onde ultrasonore de recharge réfléchie par le dispositif médical implantable, et
- d'un signal EDG représentatif des fronts montant du signal d'enveloppe REC.

Dans cet exemple, le signal d'excitation émis par le générateur 203, et par conséquent le signal ultrasonore émis par le transducteur 201, est modulé en fréquence, c'est à dire que la fréquence d'émission varie, par exemple de façon continue, par exemple de façon linéaire, entre une fréquence fₛₜₐᵣₜ et une fréquence fₛₜₒₚ, par exemple supérieure à fₛₜₐᵣₜ, entre le début et la fin de chaque impulsion.

Après chaque impulsion, une partie de l'onde ultrasonore émise par le transducteur 201 est réfléchie vers le transducteur. Un signal retour est ainsi généré aux bornes du transducteur 201. Ce signal retour est amplifié par le circuit d'amplification 205. Le signal REC correspond à l'enveloppe du signal retour amplifié. Le circuit de traitement 207 comprend par exemple un circuit analogique de détection d'enveloppe, adapté à générer le signal REC à partir du signal retour amplifié fourni par le circuit d'amplification 205.

Comme l'illustre la figure 3, le signal retour présente un retard DEL par rapport au signal d'excitation EXC, correspondant au temps de propagation aller-retour de l'onde entre le transducteur 201 et le dispositif médical implantable.

Le signal REC est représentatif de l'énergie de la portion de l'onde ultrasonore de recharge réfléchie par le dispositif médical implantable. Comme cela ressort de la figure 3, pour chaque impulsion, le signal REC présente un creux ou minimum local, correspondant à un pic d'absorption du signal ultrasonore par le dispositif implantable, à une fréquence comprise entre les fréquences fₛₜₒₚ et fₛₜₐᵣₜ. Ce minimum correspond à un mode de résonance ou d'antirésonance du dispositif médical implantable (mode 100A ou 100B de la figure 1), pour lequel le rendement de conversion d'énergie est optimal.

Pour chaque impulsion du signal ultrasonore réfléchie par le dispositif médical implantable, le signal EDG comprend un premier front montant correspondant au début de l'impulsion, et un deuxième front montant correspondant à la fin du creux du signal REC. Le circuit de traitement 207 comprend par exemple un circuit de détection de front adapté à générer le signal EDG à partir du signal REC.

A titre d'exemple, le circuit de traitement 207 est adapté à analyser le signal EDG et en déduire la fréquence f_{NZR} à laquelle la réflexion de l'onde ultrasonore par le dispositif médical implantable est minimale.

Le circuit de traitement 207 est par exemple adapté à ajuster les fréquences de début fₛₜₐᵣₜ et de fin fₛₜₒₚ du signal impulsionnel modulé, de façon à les rapprocher de la fréquence f_{NZR} et ainsi réduire l'énergie ultrasonore réfléchie par le dispositif médical implantable, et par conséquent augmenter le rendement de la chaîne de transmission d'énergie électrique au dispositif médical implantable.

A titre de variante, le signal d'excitation émis par le générateur 203, et par conséquent le signal ultrasonore émis par le transducteur 201, présente une fréquence f fixe pendant toute la durée de chaque impulsion. Dans ce cas, le circuit de traitement 207 peut être configuré pour faire varier la fréquence f entre deux impulsions, de façon à rechercher à faire tendre vers une valeur minimale l'énergie acoustique réfléchie par le dispositif médical implantable, par exemple en se basant sur un principe d'asservissement par boucle à verrouillage de phase (ou en anglais PLL : « Phase-Locked Loop »).

Dans une autre variante, l'onde ultrasonore émise par le transducteur 201 peut être une onde continue, et non une onde impulsionnelle. Dans ce cas, comme dans l'exemple décrit ci-dessus en relation avec la figure 3, l'onde ultrasonore peut être une onde modulée en fréquence, c'est à dire que sa fréquence varie de façon continue, par exemple de façon linaire, entre une fréquence fₛₜₐᵣₜ et une fréquence fₛₜₒₚ. Le cycle de variation entre les fréquences fₛₜₐᵣₜ et fₛₜₒₚ, aussi appelé cycle de modulation, peut avoir une durée constante. De façon similaire à ce qui a été décrit en relation avec la figure 3, a chaque cycle, le circuit de traitement 207 détecte la fréquence à laquelle l'énergie acoustique réfléchie par le dispositif médical implantable est minimale, et ajuste en conséquence les fréquences fₛₜₐᵣₜ et fₛₜₒₚ pour le cycle suivant.

A titre de variante, l'onde ultrasonore continue émise par le transducteur 201 présente une fréquence f fixe (non modulée), ajustable. Dans ce cas, le circuit de traitement 207 peut être configuré pour faire varier la fréquence f au cours de temps de façon à rechercher à faire tendre vers une valeur minimale l'énergie acoustique réfléchie par le dispositif médical implantable.

Bien que l'on ait décrit, en relation avec la figure 3, une solution de traitement analogique du signal retour généré par le transducteur 201, les modes de réalisation décrits ne se limitent pas à ce cas particulier.

A titre de variante (non représentée), la boucle de rétroaction du dispositif 200 peut comprendre un convertisseur analogique-numérique, par exemple en sortie de l'amplificateur 205, adapté à numériser le signal retour représentatif de l'onde réfléchie par le dispositif médical implantable. La détection de la fréquence pour laquelle la réflexion est minimale peut alors être mise en oeuvre par un circuit de traitement numérique, comprenant, par exemple, un microcontrôleur. Ceci permet la mise en oeuvre d'algorithmes de rétroaction plus complexes.

Divers modes de réalisation et variantes ont été décrits. La personne du métier comprendra que certaines caractéristiques de ces divers modes de réalisation et variantes pourraient être combinées, et d'autres variantes apparaîtront à la personne du métier. En particulier, bien que l'on ait décrit ci-dessus uniquement un exemple d'application à la recharge sans fil, par ultrasons, d'un dispositif médical implantable, les modes de réalisation décrits ne se limitent pas à ce cas particulier. Plus généralement, les modes de réalisation décrits peuvent s'appliquer à tout système de recharge sans fil, par ultrasons, d'un dispositif électronique, médical ou non, et implantable ou non.

## Revendications

1. Dispositif (200) d'émission d'ultrasons pour la recharge sans fil d'un dispositif électronique, le dispositif d'émission comprenant au moins un transducteur ultrasonore (201), et un circuit électronique (203, 205, 207) de contrôle dudit au moins un transducteur ultrasonore (201), le circuit électronique de contrôle (203, 205, 207) étant configuré pour :
- appliquer audit au moins un transducteur ultrasonore (201) un signal électrique d'excitation (EXC) de façon à provoquer l'émission, par ledit au moins un transducteur ultrasonore (201), d'une onde ultrasonore en direction du dispositif électronique ;
- lire un signal électrique retour généré par ledit au moins un transducteur ultrasonore (201) sous l'effet d'une portion de ladite onde ultrasonore réfléchie par le dispositif électronique ;
- générer un signal représentatif de l'énergie de la portion réfléchie de l'onde ultrasonore,
le circuit électronique de contrôle (203, 205, 207) comprenant une boucle de rétroaction (205, 207) configurée pour ajuster la fréquence de ladite onde ultrasonore de façon à faire tendre vers une valeur minimale l'énergie de ladite portion réfléchie de l'onde ultrasonore.

2. Dispositif (200) selon la revendication 1, dans lequel le circuit électronique de contrôle (203, 205, 207) est configuré pour appliquer audit au moins un transducteur ultrasonore (201) un signal électrique d'excitation (EXC) modulé en fréquence dont la fréquence varie de façon continue entre une fréquence fₛₜₐᵣₜ et une fréquence fₛₜₒₚ, supérieure à fₛₜₐᵣₜ.

3. Dispositif (200) selon la revendication 2, dans lequel la boucle de rétroaction (205, 207) comprend un circuit de traitement (207) adapté à détecter la fréquence f_{NZR} à laquelle l'énergie de ladite portion réfléchie de l'onde ultrasonore est minimale.

4. Dispositif (200) selon la revendication 3, dans lequel le circuit de traitement (207) est en outre configuré pour ajuster les valeurs des fréquences fₛₜₐᵣₜ et fₛₜₒₚ de façon à les rapprocher de la fréquence f_{NZR}, et ainsi réduire l'énergie de la portion réfléchie de l'onde ultrasonore.

5. Dispositif (200) selon l'une quelconque des revendications 1 à 4, dans lequel le circuit de contrôle (203, 205, 207) comprend un circuit de détection de l'enveloppe dudit signal électrique retour généré par ledit au moins un transducteur ultrasonore (201).

6. Dispositif (200) selon la revendication 5, dans lequel le circuit de contrôle (203, 205, 207) comprend un circuit de détection de fronts de ladite enveloppe dudit signal électrique retour généré par ledit au moins un transducteur ultrasonore (201).

7. Dispositif (200) selon l'une quelconque des revendications 1 à 6, dans lequel le circuit électronique de contrôle (203, 205, 207) est configuré pour appliquer audit au moins un transducteur ultrasonore (201) un signal électrique d'excitation (EXC) impulsionnel.

8. Dispositif (200) selon l'une quelconque des revendications 1 à 6, dans lequel le circuit électronique de contrôle (203, 205, 207) est configuré pour appliquer audit au moins un transducteur ultrasonore (201) un signal électrique d'excitation (EXC) continu.

9. Dispositif (200) selon l'une quelconque des revendications 1 à 8, dans lequel ledit au moins un transducteur ultrasonore (201) comprend un transducteur d'émission et un transducteur de réception.

10. Dispositif (200) selon l'une quelconque des revendications 1 à 8, dans lequel ledit au moins un transducteur ultrasonore (201) comprend un transducteur unique pour l'émission de ladite onde ultrasonore et pour la réception de ladite portion réfléchie de ladite onde ultrasonore.
